# EUROPEAN PATENT SPECIFICATION

(11) **EP 3 769 729 B1**
(45) Date of publication and mention of the grant of the patent: **21.08.2024**
(21) Application number: 19206855.9
(22) Date of filing: 04.11.2019
(51) Int. Cl.: A61F 9/00, A61M 5/42

(54) **AN INTRAOCULAR ADMINISTRATION DEVICE FOR INTRAOCULAR ADMINISTRATION OF A SUBSTANCE, FOR EXAMPLE A MEDICATION, INTO A HUMAN OR ANIMAL EYE**
VORRICHTUNG ZUR INTRAOKULAREN VERABREICHUNG ZUR INTRAOKULAREN VERABREICHUNG EINER SUBSTANZ, ZUM BEISPIEL EINES MEDIKAMENTS, IN EIN MENSCHLICHES ODER TIERISCHES AUGE
DISPOSITIF D'ADMINISTRATION INTRA-OCULAIRE D'UNE SUBSTANCE, PAR EXEMPLE D'UN MÉDICAMENT, DANS UN OEIL HUMAIN OU ANIMAL

(30) Priority: 26.07.2019 NL 2023576
(43) Date of publication of application: 27.01.2021
(73) Proprietor: Sharpsight B.V., 6049 MG Herten (NL)
(72) Inventor: GONÇALVES, Arnaldo, 6049 MG HERTEN (NL)
(74) Representative: Algemeen Octrooi- en Merkenbureau B.V.

(56) References cited:
- WO-A1-2008/097072
- WO-A1-2016/083669

## Description

The present invention relates to an intraocular administration device for intraocular administration of a substance, for example a medication, into a human or animal eye by means of a hypodermic needle according to the preamble of claim 1.

In ophthalmology, for example, intravitreal or intraocular injections by which a substance, and more particularly a medication, is injected into a human or animal eye by means of a hypodermic needle, are nowadays more and more applied for treating certain eye disorders, which until now could hardly be therapeutically treated, or only to a limited extent. Especially in the treatment of endophtalmitis, an infrequently occurring intraocular inflammation of the eye, antibiotics are often administered intravitreally. Other eye disorders that can thus be treated include: macula degeneration, vena occlusions, diabetes retinopathy, all kinds of macula oedema, neovascular glaucoma, some forms of ischemic eye disorders, etc.

In general, the hypodermic needle is to be inserted into the eye at a predetermined distance from the limbus. In particular a hypodermic needle that is inserted into the eye at an incorrect position may cause unwanted complications, such as intraocular haemorrhage, or needle damage to the eye lens, which may in turn cause cataracts, retinal detachment and the like.

As a solution to the above illustrated drawbacks, the International patent publication no. WO2008/097072 proposes a disposable device or tool for positioning at the human or animal eye, by which intravitreal injection is simplified in that the device or tool comprises a funnel shaped receptacle for receiving a hypodermic needle. The funnel shaped receptacle has an end aperture or apertures positioned such that when the tool or device is correctly positioned at the eye, the needle will be inserted into the eye at the correct position.

Similarly, the International patent publication no. WO2016/083669 proposes a disposable device or tool for positioning at the human or animal eye, by which intravitreal injection is performed in a similar manner as in WO2008/097072 as the device or tool also implements a funnel shaped receptacle for receiving a hypodermic needle.

Hospitals and other medical or clinical institutions and veterinarians nowadays apply different types of needles and syringes, which due to their diversity in dimensions are not all usable with a single known injection device or tool, such that a plurality of differently sized types of the known device have to be kept in stock, for example.

The object of the present invention is to provide an improved device or tool for assisting in the intraocular administration of a substance into the human or animal eye, suitable for use with a wide variety of different types and sizes of hypodermic needles and syringes or the like.

The above-mentioned and other objects are achieved by an intraocular administration device according to the present invention, as defined by the characterizing part of claim 1.

Different from the known device, instead of a funnel shaped receptacle for receiving a hypodermic needle or the like for administering a substance, in the device according to the present invention, in the hollow body an open access section or area is arranged, extending from the base structure, for receiving and placing the needle or syringe, such to accommodate a wide variety of types and dimensions of needles, while the open access area at the same time provides for an adequate visual inspection during administration.

To prevent the eyelashes or dirt in the eyelashes from entering the open access area, the present invention provides for wing shaped elements flaring out over a distance in radial direction from the base structure and the hollow body and extending in longitudinal direction of the hollow body on both sides of the open access area or section.

The wing shaped elements further provide for an effective spreading of the eyelids and optionally may have a curved shape to avoid injuries or the like to the eyelids and to keep the eye open in a simple and patient-friendly manner. Thus, the eyelid can be kept open in an effective manner, so that the patient's involuntary blinking his or her eyes will not interfere with the insertion of the hypodermic needle. Hence, no separate spreading elements or the like have to be applied for keeping the eye open while administering a substance, such as a medical drug.

In a further embodiment of the intraocular administration device according to the present invention, the base structure comprises an interrupted elliptical shape having an opening in the base structure connecting to the open access section, wherein the positioning means comprise at least one marker defining at least one administration position, in particular a first and second marker defining administration positions at a distance of 3.5 mm and 4 mm, respectively, from the corneal limbus of the eye.

In another embodiment said base structure comprises an elongated opening or an elliptical opening or a circular opening in said base structure, and said positioning means comprise at least one marker, defining at least one administration position, in particular a first and second marker at a distance of 3.5 mm and 4 mm, respectively, from said corneal limbus of said eye.

In these embodiments, the open access section in the hollow body and the opening or cut-off in the base structure connect to each other, such to provide a completely open and freely accessible area for administering a substance into the eye. To support the positioning of the needle or syringe at the correct position or distance from the limbus, at least one marker is provided, marking an administration position. In practice, administration positions at a distance of 3.5 mm and 4 mm from the corneal limbus of the eye are preferred.

Accordingly, a particular embodiment of the device according to the invention comprises a first and second marker defining administration positions at a distance of 3.5 mm and 4 mm, respectively, from the corneal limbus of the eye.

The marker or markers may be placed, for example, at one or both of the wing shaped elements at the sides thereof facing each other, such that the markers are visible when looking at the open access section or area.

In another embodiment according to the present invention, the positioning means of the intraocular administration device comprise a positioning element formed as an extension disposed at an interrupted part of the elliptical shaped base structure, the positioning element defining at least one administration position, in particular the positioning element defining a first and a second administration position at distance of 3.5 mm and 4 mm, respectively, from the corneal limbus of the eye.

In an embodiment of the intraocular administration device, the positioning element has an annular shaped configuration, comprising an inner annular edge defining a distance of 3.5 mm from the corneal limbus of the eye, and a recess extending in radial direction of the base structure, defining a distance of 4 mm from the corneal limbus of the eye.

The recess may have any shape, in a particular embodiment, however, the recess is arranged for accommodating hypodermic needles of 27, 30, 31 and 33 gauge, for example.

In another embodiment of the intraocular administration device according to the present invention, the wing shaped elements are tapered towards the base structure, thereby avoiding as much as possible tissue contact with the eye and its surrounding area, hence reducing irritation and trauma to the eye and tissue.

In a particular embodiment, of the intraocular administration device, a radial length of the wing shaped elements near the base structure ends at an outer annular edge of the base structure.

For the same purpose of reducing tissue contact, in a further embodiment of the intraocular administration device according to the present invention, the base structure is formed of at least three intermitted elliptical oriented support element parts.

With this embodiment, the support element is not in contact over the whole elliptical configuration of the support surface with the eye, which not only reduces tissue contact and irritation but makes it easier to place the device even in an eye with a very small eyelid crevice. Additionally, the intermittent elliptical configuration of the base structure, being formed of the at least three elliptical oriented support element parts, allows for a better visual overview of the eye region as the support element no longer obscures the eye region wholly.

In another embodiment the intermittent elliptical configuration is formed of at least four elliptical oriented support element parts. The elliptical oriented support element parts may be positioned at equidistant positions along the elliptical configuration.

Furthermore, at least one of the support element parts may be provided with friction-increasing means so as to realize a stable position on the eye. Such friction-increasing means may consist of one or more barbs provided on the at least one elliptical oriented support element part. In either example, the device is prevented from slipping away on the eyeball during handling by a doctor, a physician or a veterinarian whilst intraocular administering a substance, for example a medication or drug, into a human or animal eye.

In another example at least one of the elliptical oriented support element parts has an arc length, which differs from the arc lengths of the other elliptical oriented support element parts, whereas in yet another more simple example the elliptical oriented support element parts have identical arc lengths.

In an embodiment of the intraocular administration device according to the present invention, the longitudinal length of the wing shaped elements corresponds to a longitudinal length of the open access section of the circumferential wall in longitudinal direction of the hollow body. In particular, the longitudinal length of the wing shaped elements may exceed a longitudinal length of the open access section of the circumferential wall in longitudinal direction of the hollow body. This to effectively protect the open access section against the entrance of contaminations while administering a substance.

In an embodiment of the intraocular administration device according to the present invention the elliptical base structure has a circular shaped configuration.

The elliptical base structure may have an annular shaped configuration, such that the base structure serves as a circumferential support flange of the hollow body, while the outer or external face of the support flange may form an obtuse angle with the outer or external face of the hollow body.

In an embodiment of the intraocular administration device according to the present invention comprising an annular elliptical configuration of the base structure, the annular opening of the base structure has a size such that the inner annular edge of the base structure corresponds to, i.e. coincides with the corneal limbus of the eye.

In this embodiment, the above-mentioned distances or positions for administering a substance into the eye are measured or calculated from the annular edge of the base structure.

In a further embodiment of the present invention, in order to obtain an excellent reproducibility of successive administrations, the support element is provided with at least one orientation projection to be oriented against the edge of the limbus of the eye. Such orientation projection may be designed as a lip shaped element extending radially from the base structure, for example.

In an embodiment of the intraocular administration device according to the present invention wherein the positioning element has an annular shaped configuration, a recess is provided extending in radial direction of the base structure, the recess is V-shaped, such that the tale point of the V-shape serves as an orientation projection for placing the support element at the eye.

The invention will now be explained in more detail with reference to nonlimiting exemplary embodiments, as shown in the attached drawings, in which:
Figure 1 illustrates a cross section of an eye of a human being;
Figure 2 shows, in a perspective view, an embodiment of a device according to the present invention.
Figure 3 shows a top view of the device according to Figure 1.
Figure 4 shows, in a perspective view, a further embodiment of a device according to the present invention.
Figure 5 shows, in a perspective view, a cross section along the line V-V in Figure 4.
Figure 6 shows, in a perspective view, a yet further embodiment of a device according to the present invention.
Figure 7 shows, in a perspective view, another embodiment of the device according to Figure 4.
Figure 8 shows a front view of an embodiment of the device according to the present invention, illustrating administering distances.
Figure 9 shows a top view of another embodiment of the device according to the present invention.

In the drawings, like reference numerals denote identical or functionally identical elements.

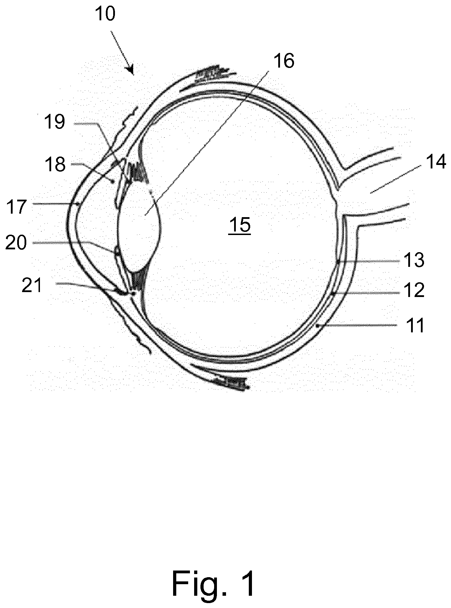

Figure 1 depicts a cross section of a human eye 10. The eye 10 is a sense organ capable of vision having an almost spherical configuration. Seen in the direction of light impinging on the eye, the anterior or frontal side of the eye 10 is formed by the cornea 17 having a stronger curvature compared to the posterior side, being the sclera 11, from which the optic nerve 14 leaves the eye 10 at its posterior side towards the brain. The limbus connects the cornea 17 and the sclera 11. The cornea 17, the iris 20 and the lens capsule 16 form the anterior chamber 18 filled with aqueous humor liquid. Behind the lens 16 the vitreous body 15 is filled with vitreous gel.

The lens 16 is suspended in the eye by means of the ciliary body 21 composed of ciliary muscle and fibers. Reference numeral 19 denotes the posterior chamber present between the lens 16, the ciliary body 21 and the iris 20. The fundus or area opposite to the lens 16 at the posterior side of the eye 10 includes the macula 13, as well as the retina, being the innermost tissue layer containing the rod and cone vision cells. Reference numeral 12 denotes the choroid.

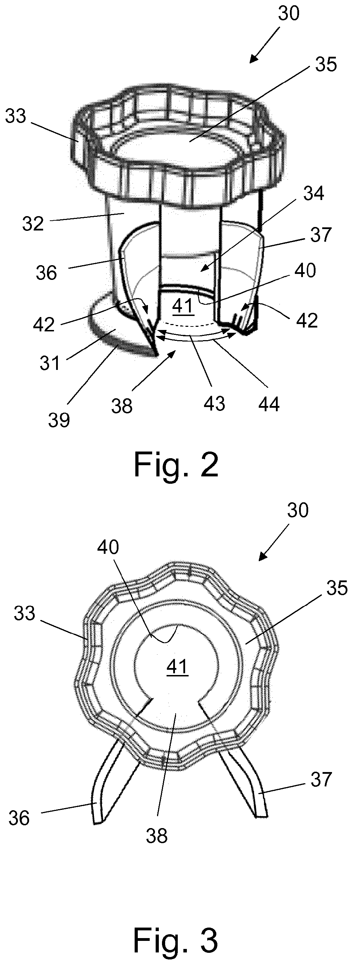

Figure 2 shows a first embodiment of a support element 30 suitable for intraocular administration of a substance into the human or animal eye. The support element 30 comprises an elliptical annular shaped base structure 31. From the base structure 31 a longitudinal, cylindrical, open hollow body 35 extends, having an upright circumferential wall 32 that extends over a distance transverse to the base structure 31. The upright circumferential wall 32 of the open hollow body 35 ends in a gear shaped grip 33, forming an ergonomic grip for firmly gripping and manipulating, i.e. positioning of the support element at the eye 10 by hand. The hollow body 35 may have a form or shape that slightly tapers outwardly near the end comprising the grip 33, this to provide a sufficiently large gripping area. Those skilled in the art will appreciate that other types of ergonomic grips may be provided.

The annular shaped configuration of the elliptical base structure 31 provides a support surface serving as a circumferential support flange of the support element 30 for placement on the eye. The outer or external face of the support flange 31 forms an obtuse angle with the outer or external face of the upright wall 32 of the hollow body 35.

The upright circumferential wall 32 comprises an open access section 34, i.e. open to the interior of the hollow body 35, extending from the base structure 31 in longitudinal direction of the hollow body 35. The access section 34 defines an open area having a width and height sufficiently for administration of a substance from the access section 34 into the eye, while providing visual inspection of the administration of the substance by a doctor, a physician or a veterinarian when applying same to the eye. In the embodiment shown, the access section has a height of about half the length of the circumferential wall 32 and a width or arc length of about one sixth of the circumference of the wall 32. Instead of a rectangular shaped access section, an elliptical, circular or otherwise shaped access opening may be provided.

In the embodiment of the support element 30, part 38 of the annular base structure or flange 31 connecting to the access section 34 is cut away, i.e. interrupted or open and connects to the open access section 34.

On each longitudinal side of the access section or opening 34 a wing shaped spreading element 36, 37 extends over a distance in radial direction from the base structure 31 and extends from the base structure 31 in longitudinal direction of the upright circumferential wall 32 of the hollow body 35. The wing shaped spreading elements 36, 37 are at least spaced apart over the circumferential width or arc length of the access section 34.

In the embodiment shown, the wing shaped spreading elements 36, 37 flair outwardly from the circumferential wall 32 and have a curved shape bending towards each other. The wing shaped elements 36, 37 are tapered towards the base structure 31, that is the distance over which the wing shaped elements 36, 37 extend in radial direction from the circumferential wall 32 reduces in the direction of the base structure 31. Thereby avoiding as much as possible tissue contact with the eye and its surrounding area, hence reducing irritation and trauma to the eye and tissue.

In the embodiment of the support element 30, the radial length of the wing shaped elements 36, 37 near the base structure 31 ends at an outer annular edge 39 of the base structure 31. The wing shaped elements 36, 37 have a longitudinal length exceeding the length of the open access section 34. However, it may be sufficient for the wing shaped elements 36, 37 to just cover the length of the access section 34 in longitudinal direction of the wall 32. This to effectively protect the open access section 34 against the entrance of contaminations while administering a substance.

The annular opening 41 of the base structure 31 has a size such that the inner annular edge 40 of the base structure 31 corresponds to, i.e. coincides with the corneal limbus of the eye 10 when the support element is positioned at or into the eye. For clarity purposes, the inner annular edge 40 is partly shown by a dashed line.

As can be seen from Figure 1, in the support element 30, the open access section 34 in the circumferential upright wall 32 and the opening or cut-off 38 in the base structure 31 provide a completely open and freely accessible area for administering a substance into the eye. To support the positioning of the needle or syringe at the correct position or distance from the limbus, each of the wing shaped elements 36, 37 at the inner sides thereof facing each other, is provided with markers 42, marking an administration position. Such as markers in the form of printed lines and/or ridges extending from the respective surface and/or carves in the surface of the wing shaped elements 36, 37.

The wing shaped elements 36, 37 may be made of a plastic material, for example, such that a doctor or physician may indicate himself or herself a suitable marking at a wing surface, such as by scratching or printing a mark, after measuring the human eye. In this way, a very versatile support element is provided, capable of supporting nearly all types of administration needles or syringes or the like.

In the embodiment 30, two markers 42 are provided on each wing shaped element, marking administration positions at a distance 43 of 3.5 mm and a distance 44 of 4 mm from the corneal limbus of the eye 10, i.e. measured in radial direction form the inner annular edge 40 of the base structure 31, respectively.

That is, when placing a needle or syringe at one of the positions 43 or 44, an administration position of 3.5 mm or 4 mm, respectively, from the corneal limbus of the eye is guaranteed.

Figure 2 shows, for clarity purposes, a top view of the support element 30 of Figure 1.

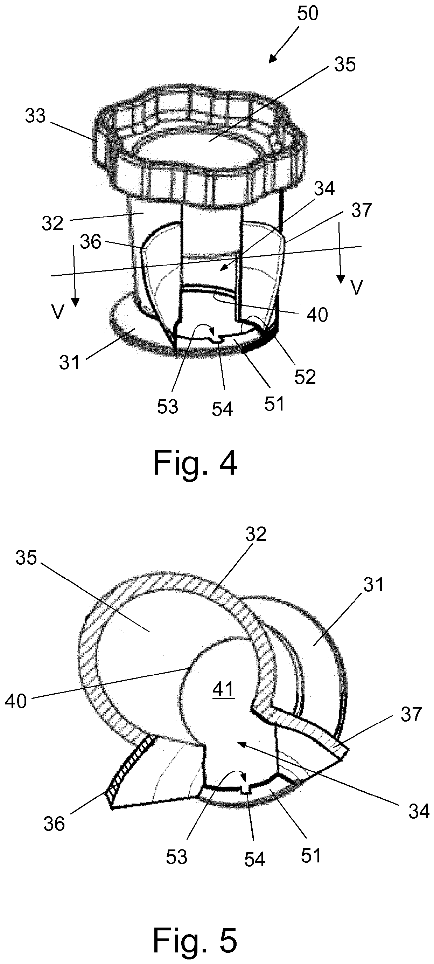

Figure 4 shows a second embodiment of a support element 50 according to the present invention, and Figure 5 shows a cross section along the line of intersection V-V in Figure 4.

In the embodiment 50, the positioning means of the intraocular administration device comprise a positioning element formed as an extension or connecting element 51 disposed at the interrupted part 38 of the elliptical shaped base structure 31 (see Figure 2). In this embodiment 50, the extension or connecting element 51 extends between and connects the two wing shaped elements 36, 37 and has an inner annular edge 52 defining a distance of 3.5 mm from the corneal limbus of the eye, i.e. a distance of 3.5 mm with respect to the inner annular edge 40 of the base structure 31 measured in outer radial direction.

The extension or connecting element 51 further comprises a rectangular recess 53 open from the inner annular edge 52 and extending in radial direction of the base structure 31. The closed edge 54 of the recess 53 defines a distance of 4 mm from the corneal limbus of the eye, i.e. a distance of 4 mm from the inner annular edge 40 of the base structure 31, measured in outer radial direction.

Those skilled in the art will appreciate that for administration purposes other radial distances may be selected, i.e. the extension or connecting element 51 may have an inner annular edge 52 placed closer to or further away from the inner annular edge 40 of the base structure 31. The extension or connecting element 51 may also have a stepped shaped inner annular edge 52, defining a plurality of administration positions, and/or several recesses 53 extending at different radial distances, for example (not shown).

The recess may have any shape, in a particular embodiment however, the recess 53 is arranged for accommodating hypodermic needles of 27, 30, 31 and 33 gauge, i.e. 0.361 mm, 0.25 mm, 0.226 mm and 0.18 mm, respectively.

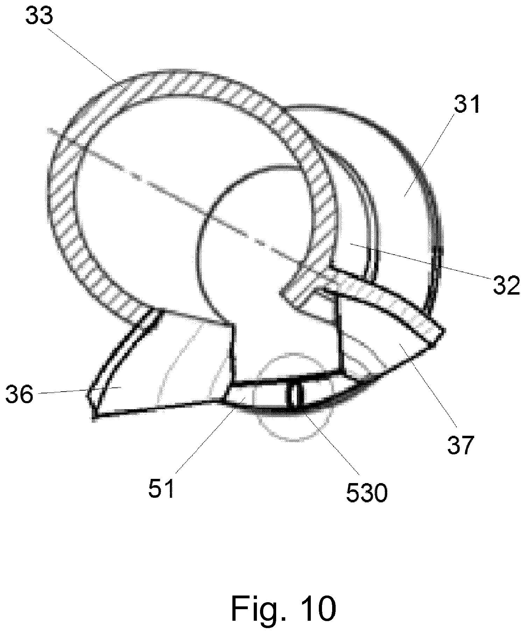

Figure 10 shows an alternative embodiment incorporating an opening 530 present in the extension or connecting element 51. The opening 530 is depicted as having an elongated shape, for example as a rectangular opening with rounded corners or as an ellipse 530. In all embodiments of the elongated shaped opening 530 its major axis extends in radial direction of the base structure 31. The elongated shaped opening 530 (here an ellipse) allows the insertion of the needle are several different radial distances from the corneal limbus of the eye, (i.e. at 3.5 mm or 4.0 mm). The opening can also be a circular opening.

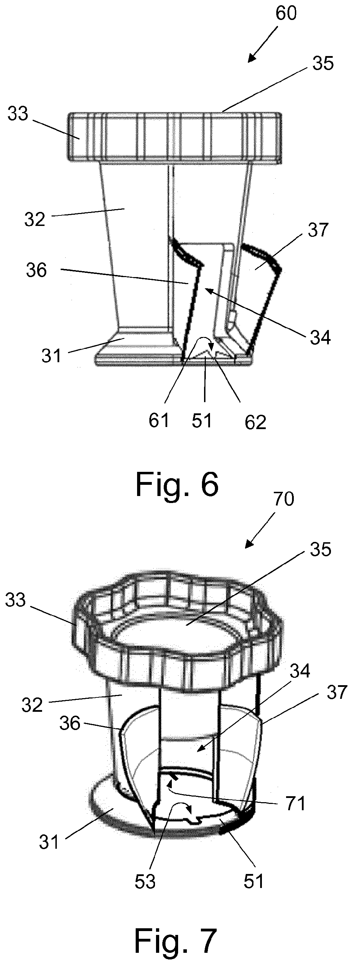

Figure 6 shows a third embodiment of a support element 60 in accordance with the present invention. In this embodiment the support element 60 comprises an extension or connecting element 51 that extends between and connects the two wing shaped elements 36, 37 and has an inner annular edge 52 defining a distance of 3.5 mm from the corneal limbus of the eye, i.e. a distance of 3.5 mm with respect to the inner annular edge 40 of the base structure 31 measured in outer radial direction, like the support element 50.

In this third embodiment, the extension or connecting element 51 of the support element 60 comprises a V-shaped recess 61 open from the inner annular edge 52 and extending in radial direction of the base structure 31. The valley or intersection point 62 of the recess 61 defines a distance of 4 mm from the corneal limbus of the eye, i.e. a distance of 4 mm from the inner annular edge 40 of the base structure 31, measured in outer radial direction.

In addition, the valley or intersection point 62 of the recess 61 serves as an orientation projection or point to be oriented against the edge of the limbus of the eye. Such orientation projection may also be designed as a lip shaped element 71 extending radially inwardly of the hollow body 35, i.e. inwardly from the base structure 31, i.e. the inner annular edge 40, for example, as shown in a fourth embodiment of the support element 70 according to the present invention, illustrated in Figure 7.

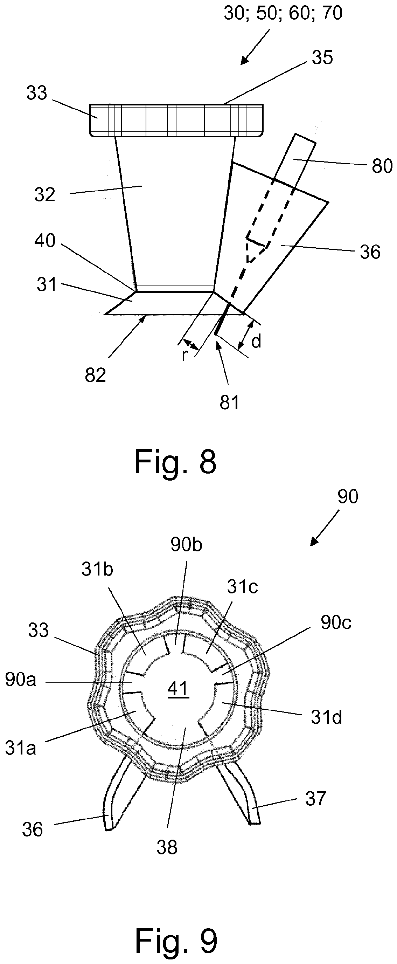

For illustrative purposes, Figure 8 shows the embodiments 30, 50, 60, 70 of the present invention in a front face, against the wing shaped element 36. With partly dashed lines 80 a hypodermic needle or syringe is illustrated, having an insertion end 81 for administering a substance, i.e. a liquid, into the eye 10. The distance r illustrates the administering distance from the limbus of eye when a support device 30, 50, 60, 70 is placed on the eye 10. Such as a distance of 3.5 mm or 4 mm, for example. The distance d illustrates an injection distance from the lower surface 82 of the base structure 31, such as a distance of 4 - 6 mm, for example.

Figure 9 illustrates a top view of a fifth embodiment of a support element 90 according to the present invention, wherein the base structure 31 exhibits an intermittent elliptical configuration being formed of four tangentially spaced apart base structure parts 31a, 31b, 31c, 31d. Because of the interrupted, intermittent elliptical configuration of the base structure 31 the contact area with the eye 10 is minimal, so that the patient is optimally protected against trauma and/or irritation.

Additionally, the creation of open access spaces 90a, 90b, 90c between the base structure parts 31a, 31b, 31c, 31d allows for a better visual overview of the region of the eye 10 as the support element no longer obscures for the greater part the region of interest of the eye 10.

As shown in Figure 9, the base structure parts 31a, 31b, 31c, 31d may have different arc lengths. Likewise, the open access spaces 90a, 90b, 90c may have different sizes. However, the base structure parts 31a, 31b, 31c, 31d and/or the open access spaces 90a, 90b, 90c may have identical arc lengths, for example. The base structure may be at least comprised of three separated base structure parts.

The base structure 31 as well as the base structure parts 31a, 31b, 31c, 31d may comprise a circular shaped configuration.

The support element of the intraocular administration device according to the present invention may be manufactured from a plastics material, in particular a plastics material suitable for therapeutical and medical purposes, and may be designed as a disposable device. The support element according to the present invention is suitable for safely and securely administering a substance, such as a medical liquid, into the human or animal eye by a large variety of hypodermic needles, syringes and the like, having different dimensions and designs.

## Claims

1. An intraocular administration device for intraocular administration of a substance, for example a medication, into a human or animal eye (10) by means of a hypodermic needle, said device comprising:
a support element (30; 50; 60; 70; 90), arranged to be placed on said eye (10), said support element comprising:
a base structure (31) having an elliptical shaped configuration for placement on said eye (10); and
positioning means for assisting administration of said substance at at least one predetermined distance from the corneal limbus of said eye, said support element further comprising:
a hollow body (35) defining an upright circumferential wall (32) extending in transverse direction from said base structure (31), said upright wall (32) comprising an open access section (34), extending from the base structure (31) and suitable for administration of said substance and for visual inspection of said administration of said substance,
**characterized in that**
said hollow body (35) further comprising spreading elements (36, 37) for spreading eyelids of said eye (10), said spreading elements (36, 37) comprising two spaced apart wing shaped elements extending in radial direction from said base structure (31) in longitudinal direction of said hollow body on both sides of said open access section (34).

2. The intraocular administration device according to claim 1, wherein said base structure (31) comprises an interrupted elliptical shape defining an opening (38) in said base structure connecting to said open access section (34), and said positioning means comprise at least one marker (42), defining at least one administration position, in particular a first and second marker at a distance of 3.5 mm and 4 mm, respectively, from said corneal limbus of said eye (10).

3. The intraocular administration device according to claim 1, wherein said base structure (31) comprises an elongated opening (530) or an elliptical opening (530) or a circular opening (530) in said base structure, and said positioning means comprise at least one marker (42), defining at least one administration position, in particular a first and second marker at a distance of 3.5 mm and 4 mm, respectively, from said corneal limbus of said eye (10).

4. The intraocular administration device according to claim 1, wherein said positioning means comprise a positioning element defined as an extension (51) disposed at an interrupted part of said elliptical shaped base structure (31), said positioning element defining at least one administration position (52, 53), in particular said positioning element defining a first (52) and a second (53) administration position at distance of 3.5 mm and 4 mm, respectively, from said corneal limbus of said eye (10).

5. The intraocular administration device according to claim 4, wherein said positioning element (51) has an annular shaped configuration, comprising an inner annular edge (52) defining a distance of 3.5 mm from said corneal limbus of said eye, and a recess (53) extending in radial direction of the base structure defining a distance of 4 mm from said corneal limbus of said eye.

6. The intraocular administration device according to claim 5, wherein said recess (53) is arranged for accommodating hypodermic needles of 27, 30, 31 and 33 gauge.

7. The intraocular administration device according to any of the previous claims, wherein said wing shaped elements (36, 37) are tapered towards said base structure (31).

8. The intraocular administration device according to any of the previous claims, wherein a radial length of said wing shaped elements (36, 37) near said base structure (31) ends at an outer annular edge (39) of said base structure.

9. The intraocular administration device according to any of the previous claims, wherein a longitudinal length of said wing shaped elements (36, 37) corresponds to a longitudinal length of said open access section (34) of said circumferential wall (32).

10. The intraocular administration device according to any of the claims 1 - 8, wherein a longitudinal length of said wing shaped elements (36, 37) exceeds a longitudinal length of said open access section (34) of said circumferential wall (32).

11. The intraocular administration device according to any of the previous claims, wherein said elliptical base structure (31) has a circular shaped configuration or an annular shaped configuration.

12. The intraocular administration device according to claim 11, wherein an inner annular edge (40) of said base structure (31) corresponds to said corneal limbus of said eye (10).

13. The intraocular administration device according to any of the previous claims, wherein said base structure (31) is formed of at least three intermitted elliptical oriented support element parts (31a, 31b, 31c).

14. The intraocular administration device according to claim 12, wherein said elliptical oriented support element parts (31a, 31b, 31c) are positioned at equidistant positions along said elliptical configuration of said base structure (31).

15. The intraocular administration device according to any of the previous claims, wherein the support element is provided with at least one orientation projection (71) to be oriented against an edge of the limbus of the eye (10).

## Patentansprüche

1. Vorrichtung zur intraokularen Verabreichung zur intraokularen Verabreichung einer Substanz, zum Beispiel eines Medikaments, in ein menschliches oder tierisches Auge (10) mithilfe einer hypodermischen Nadel, wobei die Vorrichtung umfasst:
ein Stützelement (30; 50; 60; 70; 90), das dazu angeordnet ist, an dem Auge (10) platziert zu werden, wobei das Stützelement umfasst:
eine Basisstruktur (31) mit einer elliptisch geformten Auslegung zur Platzierung an dem Auge (10); und
Positionierungsmittel zur Unterstützung der Verabreichung der Substanz in mindestens einem vorbestimmten Abstand zum Hornhautlimbus des Auges, wobei das Stützelement ferner umfasst:
einen Hohlkörper (35), der eine aufrechte Umfangswand (32) definiert, die sich in Querrichtung von der Basisstruktur (31) erstreckt, wobei die aufrechte Wand (32) einen offenen Zugangsbereich (34) umfasst, der sich von der Basisstruktur (31) erstreckt und zur Verabreichung der Substanz und zur Sichtkontrolle der Verabreichung der Substanz geeignet ist,
**dadurch gekennzeichnet, dass** der Hohlkörper (35) ferner Spreizelemente (36, 37) zum Spreizen der Augenlider des Auges (10) umfasst, wobei die Spreizelemente (36, 37) zwei beabstandete Flügelelemente umfasst, die sich in radialer Richtung von der Basisstruktur (31) in Längsrichtung des Hohlkörpers auf beiden Seiten des offenen Zugangsbereichs (34) erstrecken.

2. Vorrichtung zur intraokularen Verabreichung nach Anspruch 1, wobei die Basisstruktur (31) eine unterbrochene elliptische Form umfasst, die eine Öffnung (38) in der Basisstruktur definiert, die mit dem offenen Zugangsbereich (34) verbunden ist, und wobei die Positionierungsmittel mindestens eine mindestens eine Verabreichungsposition definierende Markierung (42) umfassen, insbesondere eine erste und eine zweite Markierung in einem Abstand von 3,5 mm bzw. 4 mm zu dem Hornhautlimbus des Auges (10).

3. Vorrichtung zur intraokularen Verabreichung nach Anspruch 1, wobei die Basisstruktur (31) eine längliche Öffnung (530) oder eine elliptische Öffnung (530) oder eine kreisförmige Öffnung (530) in der Basisstruktur umfasst, und die Positionierungsmittel mindestens eine mindestens eine Verabreichungsposition definierende Markierung (42) umfassen, insbesondere eine erste und eine zweite Markierung in einem Abstand von 3,5 mm bzw. 4 mm zu dem Hornhautlimbus des Auges (10).

4. Vorrichtung zur intraokularen Verabreichung nach Anspruch 1, wobei die Positionierungsmittel ein Positionierungselement umfassen, das als eine Ausdehnung (51) definiert ist, die an einem unterbrochenen Teil der elliptisch geformten Basisstruktur (31) angeordnet ist, wobei das Positionierungselement mindestens eine Verabreichungsposition (52, 53) definiert, wobei insbesondere das Positionierungselement eine erste (52) und eine zweite (53) Verabreichungsposition im Abstand von 3,5 mm bzw. 4 mm zu dem Hornhautlimbus des Auges (10) definiert.

5. Vorrichtung zur intraokularen Verabreichung nach Anspruch 4, wobei das Positionierungselement (51) eine ringförmig geformte Auslegung hat und eine ringförmige Innenkante (52) umfasst, die einen Abstand von 3,5 mm zu dem Hornhautlimbus des Auges definiert, und eine Aussparung (53), die sich in radialer Richtung von der Basisstruktur erstreckt und einen Abstand von 4 mm zu dem Hornhautlimbus des Auges definiert.

6. Vorrichtung zur intraokularen Verabreichung nach Anspruch 5, wobei die Aussparung (53) zur Aufnahme von hypodermischen Nadeln mit einer Gauge-Zahl von 27, 30, 31 und 33 angeordnet ist.

7. Vorrichtung zur intraokularen Verabreichung nach einem der vorhergehenden Ansprüche, wobei die flügelförmigen Elemente (36, 37) in Richtung der Basisstruktur (31) konisch zulaufen.

8. Vorrichtung zur intraokularen Verabreichung nach einem der vorhergehenden Ansprüche, wobei eine radiale Länge der flügelförmigen Elemente (36, 37) nahe der Basisstruktur (31) an einer ringförmigen Außenkante (39) der Basisstruktur endet.

9. Vorrichtung zur intraokularen Verabreichung nach einem der vorhergehenden Ansprüche, wobei eine Längslänge der flügelförmigen Elemente (36, 37) einer Längslänge des offenen Zugangsbereiches (34) der Umfangswand (32) entspricht.

10. Vorrichtung zur intraokularen Verabreichung nach einem der Ansprüche 1 - 8, wobei eine Längslänge der flügelförmigen Elemente (36, 37) einer Längslänge des offenen Zugangsbereiches (34) der Umfangswand (32) entspricht.

11. Vorrichtung zur intraokularen Verabreichung nach einem der vorhergehenden Ansprüche, wobei die elliptische Basisstruktur (31) eine kreisförmig geformte Auslegung oder eine ringförmig geformte Auslegung hat.

12. Vorrichtung zur intraokularen Verabreichung nach Anspruch 11, wobei eine ringförmige Innenkante (40) der Basisstruktur (31) dem Hornhautlimbus des Auges (10) entspricht.

13. Vorrichtung zur intraokularen Verabreichung nach einem der vorhergehenden Ansprüche, wobei die Basisstruktur (31) aus mindestens drei intermittierenden elliptisch ausgerichteten Stützelementteilen (31a, 31b, 31c) gebildet ist.

14. Vorrichtung zur intraokularen Verabreichung nach Anspruch 12, wobei die elliptisch ausgerichteten Stützelementteile (31a, 31b, 31c) an Positionen mit gleichem Abstand dazwischen entlang der elliptischen Auslegung der Basisstruktur (31) positioniert sind.

15. Vorrichtung zur intraokularen Verabreichung nach einem der vorhergehenden Ansprüche, wobei das Stützelement mit mindestens einem Ausrichtungsvorsprung (71) versehen ist, um gegen einen Rand des Limbus des Auges (10) ausgerichtet zu werden.

## Revendications

1. Dispositif d'administration intraoculaire pour l'administration intraoculaire d'une substance, par exemple un médicament, dans un oeil humain ou animal (10) au moyen d'une aiguille hypodermique, ledit dispositif comprenant :
un élément de support (30 ; 50 ; 60 ; 70 ; 90), agencé pour être placé sur ledit oeil (10), ledit élément de support comprenant :
une structure de base (31) présentant une configuration de forme elliptique pour être placée sur ledit oeil (10) ; et
des moyens de positionnement pour aider à l'administration de ladite substance à au moins une distance prédéterminée du limbe cornéen dudit oeil, ledit élément de support comprenant en outre :
un corps creux (35) définissant une paroi circonférentielle verticale (32) s'étendant dans la direction transversale à partir de ladite structure de base (31), ladite paroi verticale (32) comprenant une section d'accès ouverte (34), s'étendant à partir de la structure de base (31) et adaptée à l'administration de ladite substance et à l'inspection visuelle de ladite administration de ladite substance,
**caractérisé en ce que**
ledit corps creux (35) comprenant en outre des éléments d'écartement (36, 37) pour écarter les paupières dudit oeil (10), lesdits éléments d'écartement (36, 37) comprenant deux éléments en forme d'aile espacés s'étendant dans la direction radiale à partir de ladite structure de base (31) dans la direction longitudinale dudit corps creux des deux côtés de ladite section d'accès ouverte (34).

2. Dispositif d'administration intraoculaire selon la revendication 1, dans lequel ladite structure de base (31) comprend une forme elliptique interrompue définissant une ouverture (38) dans ladite structure de base se reliant à ladite section d'accès ouverte (34), et lesdits moyens de positionnement comprennent au moins un marqueur (42), définissant au moins une position d'administration, en particulier un premier et un second marqueur à une distance de 3,5 mm et 4 mm, respectivement, dudit limbe cornéen dudit oeil (10).

3. Dispositif d'administration intraoculaire selon la revendication 1, dans lequel ladite structure de base (31) comprend une ouverture allongée (530) ou une ouverture elliptique (530) ou une ouverture circulaire (530) dans ladite structure de base, et lesdits moyens de positionnement comprennent au moins un marqueur (42), définissant au moins une position d'administration, en particulier un premier et un second marqueur à une distance de 3,5 mm et 4 mm, respectivement, dudit limbe cornéen dudit oeil (10).

4. Dispositif d'administration intraoculaire selon la revendication 1, dans lequel lesdits moyens de positionnement comprennent un élément de positionnement défini comme une extension (51) disposé au niveau d'une partie interrompue de ladite structure de base de forme elliptique (31), ledit élément de positionnement définissant au moins une position d'administration (52, 53), en particulier ledit élément de positionnement définissant une première (52) et une seconde (53) positions d'administration à une distance de 3,5 mm et 4 mm, respectivement, dudit limbe cornéen dudit oeil (10).

5. Dispositif d'administration intraoculaire selon la revendication 4, dans lequel ledit élément de positionnement (51) présente une configuration de forme annulaire, comprenant un bord annulaire interne (52) définissant une distance de 3,5 mm dudit limbe cornéen dudit oeil, et un évidement (53) s'étendant dans la direction radiale de la structure de base définissant une distance de 4 mm dudit limbe cornéen dudit oeil.

6. Dispositif d'administration intraoculaire selon la revendication 5, dans lequel ledit évidement (53) est agencé pour recevoir des aiguilles hypodermiques de calibre 27, 30, 31 et 33.

7. Dispositif d'administration intraoculaire selon l'une des revendications précédentes, dans lequel lesdits éléments en forme d'aile (36, 37) sont effilés vers ladite structure de base (31).

8. Dispositif d'administration intraoculaire selon l'une des revendications précédentes, dans lequel une longueur radiale desdits éléments en forme d'aile (36, 37) à proximité de ladite structure de base (31) se termine à un bord annulaire externe (39) de ladite structure de base.

9. Dispositif d'administration intraoculaire selon l'une des revendications précédentes, dans lequel une longueur longitudinale desdits éléments en forme d'aile (36, 37) correspond à une longueur longitudinale de ladite section d'accès ouverte (34) de ladite paroi circonférentielle (32).

10. Dispositif d'administration intraoculaire selon l'une des revendications 1 à 8, dans lequel une longueur longitudinale desdits éléments en forme d'aile (36, 37) dépasse une longueur longitudinale de ladite section d'accès ouverte (34) de ladite paroi circonférentielle (32).

11. Dispositif d'administration intraoculaire selon l'une des revendications précédentes, dans lequel ladite structure de base elliptique (31) présente une configuration de forme circulaire ou une configuration de forme annulaire.

12. Dispositif d'administration intraoculaire selon la revendication 11, dans lequel un bord annulaire interne (40) de ladite structure de base (31) correspond audit limbe cornéen dudit oeil (10).

13. Dispositif d'administration intraoculaire selon l'une des revendications précédentes, dans lequel ladite structure de base (31) est formée d'au moins trois parties d'élément de support à orientation elliptique intermittente (31a, 31b, 31c).

14. Dispositif d'administration intraoculaire selon la revendication 12, dans lequel lesdites parties d'élément de support à orientation elliptique (31a, 31b, 31c) sont positionnées à des positions équidistantes le long de ladite configuration elliptique de ladite structure de base (31).

15. Dispositif d'administration intraoculaire selon l'une des revendications précédentes, dans lequel l'élément de support est pourvu d'au moins une saillie d'orientation (71) destinée à être orientée contre un bord du limbe de l'oeil (10).
